# EUROPEAN PATENT SPECIFICATION

(11) **EP 2 724 746 B1**
(45) Date of publication and mention of the grant of the patent: **10.12.2014**
(21) Application number: 12190370.2
(22) Date of filing: 29.10.2012
(51) Int. Cl.: A61N 5/06

(54) **System and method for scheduling light therapy**
System und Verfahren zur Planung einer Lichttherapie
Système et procédé de programmation de thérapie lumineuse

(43) Date of publication of application: 30.04.2014
(73) Proprietor: Valkee Oy, 90590 Oulu (FI)
(72) Inventor: Nissilä, Juuso, 91100 Ii (FI); Jurvelin, Heidi, 90240 Oulu (FI)
(74) Representative: Høiberg A/S

(56) References cited:
- WO-A1-2008/029001
- WO-A1-2012/130958
- WO-A2-2004/110305
- KOLLA, B AND AUGER, R.: "Jet lag and shift work sleep disorders:How to help reset the internal clock", CLEVELAND CLINIC JOURNAL OF MEDICINE, vol. 78, no. 10, 1 October 2011 (2011-10-01), pages 675-684, XP002693915,

## Description

The present invention relates to a computer implemented method for scheduling light therapy to prevent symptoms of jetlag when travelling across multiple time zones. The invention further relates to a system for preventing symptoms of jetlag of a user travelling across multiple time zones.

### Background of invention

Jetlag is a physiological condition which results from alterations to the body's circadian rhythms resulting from rapid long-distance travelling. When traveling across a number of time zones, the body clock will be out of synchronization with the destination time, as it experiences daylight and darkness contrary to the rhythms to which it has grown accustomed Jetlag is thus a consequence of difference in internal and external circadian rhythms. The condition of jetlag may last several days until one is fully adjusted to the new time zone. The duration of jetlag depends on the number of time zones crossed, direction of travel, time of day of the flight, and possibly time of the year (Arendt 2009). A recovery rate of one day per time zone crossed is a suggested guideline; however crossing one or two time zones typically does not cause jetlag. Disturbance in circadian rhythm impacts both physical and mental health (Winget et al. 1984). Symptoms of jetlag include for example poor sleep quality at night, drowsiness during the day, reduced alertness, reduced physical and cognitive performance, exhaustion, irritation, depression and digestive complications (Waterhouse et al. 2007). Reduction of physical performance due to jetlag has been observed in top athletes (Recht et al. 1995). The symptoms of jetlag are alleviated as the internal clock shifts gradually towards the external time (Eastman et al. 2005, Haimov & Arendt 1999), but the speed at which the body adjusts to the new schedule depends on the individual; some people may require several days to adjust to a new time zone, while others experience little disruption. Individual variation in how jetlag is subjectively perceived is therefore observed. To date there is no clear explanation for the individual differences. It is possible that younger people with flexible sleep rhythm and better physical shape experience fewer jetlag symptoms (Reilly et al. 2009). There are also opposite findings about the significance of age to jetlag symptoms. In addition, also morning - evening typicality and congenital tolerance may influence perceived jet lag symptoms. (Auger & Morgenthaler 2009).

It is believed that bright light therapy administered through the eyes, e.g. in the form of bright light lamps placed in front of the face, can speed up this adjustment process. The purpose of this form of bright light therapy is to accelerate reconciliation of internal and external circadian phases (Auger & Morgenthaler 2009). Bright light therapy has been shown in several controlled studies to boost shifting of circadian rhythm and thereby to alleviate the symptoms of jetlag (Waterhouse et al. 2007, Eastman et al. 2005, Arendt 2009). The drawback of this conventional light therapy is that the amount of light required may be so high that delivering it via the ocular route may cause damage to the eye nerve, headache and other harmful side effects. Another drawback is that the recommended treatment time is at least half an hour, and preferably at least one hour, which limits a person's daily life. The person should also be very close to the light device to realize a therapeutic effect, preferably as close as 30-50 cm, which makes administration cumbersome. Traditional light therapy lamps also must produce 2,500-10,000 lux, making these light units very high in energy consumption. WO 98/51372 discloses a method of resetting the circadian clock by applying non-solar photic stimulation of 15 to 150,000 lux, preferably 10,000 to 13,000 lux to any non-ocular region of the human body for 15 minutes to about 12 hours, preferably for 3 hours. Such treatment is hard to carry out without affecting normal activity. Recently a more elegant solution has been commercialized in the form of the Valkee Brain Stimulation Headset described in WO 2008/029001, wherein LED (Light Emitting Diode) bright light is provided to the brain through the auditory canal.

### Summary of invention

Knowing that bright light therapy can shift the circadian rhythm the question is then how to apply the therapy in the right dose in the right way to effectively prevent the symptoms of jetlag when travelling across time zones. Timing of treatment, as well as intensity and spectrum of the light, impact the magnitude of the shift (Arendt 2009). "Jetlag calculators" are currently available to find out when and how to use bright light lamps, and when light should be avoided. However, bright light therapy administered non-ocularly, e.g. through the auditory canal, is more pleasant and more effective. One purpose of the invention is therefore to determine when to apply the therapy when travelling.

One embodiment of the invention therefore discloses a computer implemented method for calculating a schedule of bright light treatments for preventing symptoms of jetlag when crossing a number of time zones during travelling, the method comprising calculating a schedule of doses of bright light beginning at the day of arrival, each dose separated by an interval of between 2 and 4 hours, such as 3 hours, wherein when travelling west the time of the first dose at the day of arrival is scheduled during the forenoon between 11 and 13 o'clock, such as 12 o'clock, and when travelling east the time of the first dose at the day of arrival is scheduled at around x+7 o'clock if crossing less than six time zones, and at around x o'clock if crossing at least six time zones, where x is the number of time zones crossed.

The method may be implemented directly in the Valkee Brain Stimulation Headset. Or the method may be effectuated from a website or it may be integrated in a personal computer, mobile phone, smartphone or other electronic device capable of executing computer code.

In healthy adults, body temperature fluctuates about 0.5°C throughout the day, with lower temperatures in the morning and higher temperatures in the late afternoon and evening, as the body's needs and activities change. Thus, the body temperature cycles regularly up and down through the day, as controlled by the person's circadian rhythm. The core body temperature of an individual tends to have the lowest value in the second half of the sleep cycle. This minimum value, the time of daily temperature minimum Tₘᵢₙ (also called the nadir), is one of the primary markers for circadian rhythms. The time of daily temperature minimum Tₘᵢₙ occurs between 3.30 and 5.00 am, typically around 4 am, i.e. about 2-3 hours before the person normally wakes up (assuming the person sleeps at night and stays awake during the day). Also physical and mental performance seems to follow the circadian rhythm (Reilly et al. 2005; Reilly & Waterhouse 2009). Typically performance is strongest in late afternoon / early evening when the body temperature is at its highest.

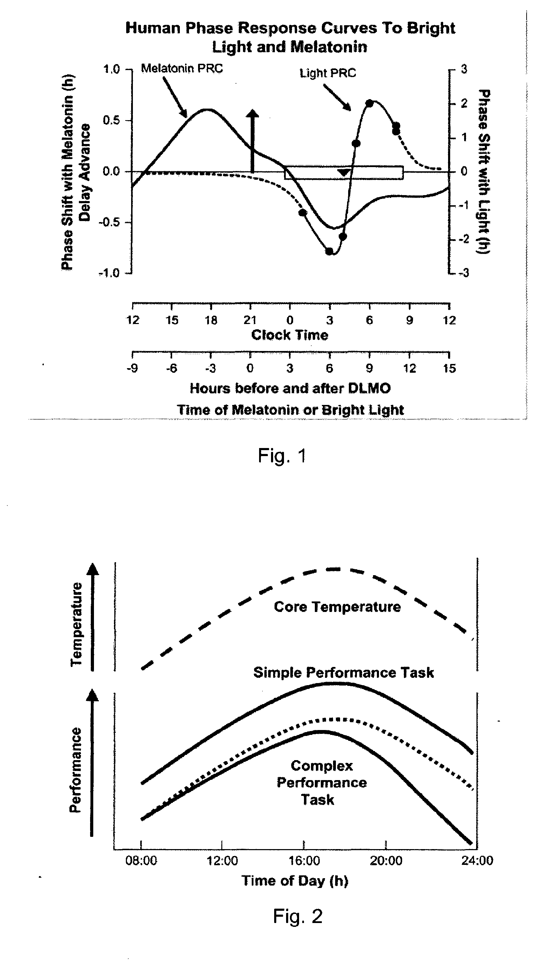

As jetlag is at least partly caused by the shifting of the circadian rhythm, alleviation of jetlag symptoms is therefore primarily based on resetting the bodily internal clock by a controlled phase shift of the circadian rhythm by correctly timed and dosed bright light treatments. The present invention is based on the observation that the timing of treatment impacts the magnitude of the shift (Arendt 2009). The magnitude of the phase shift also depends on the intensity of the light (Reilly et al. 2009). The so called phase response curve (PRC) of light (see fig. 1) can be used to determine the connection between timing of the light stimulus and the magnitude of the phase shift at different phases of the circadian rhythm (Khalsa et al. 2003). The advancing or delaying of the phase is connected to the time of the daily temperature minimum Tₘᵢₙ and the timing of light exposure. If the light exposure occurs before temperature Tₘᵢₙ, the phase tends to be delayed, i.e. moved forward in time. On the other hand, light exposure after Tₘᵢₙ causes the phase to advance (Eastman et al. 2005), i.e. moved backwards in time. Based on the light response curve the greatest phase shift can be obtained when light exposure is administered about 3 hours before Tₘᵢₙ (when delaying the phase shift) or about 3 hours after Tₘᵢₙ (when advancing the phase shift) (Khalsa et al. 2003). It can be further deduced from the phase response curve that phase shifts can only be obtained within a window of plus/minus approx. 6 hours of Tₘᵢₙ, i.e. when delaying the circadian rhythm, light exposure should be administered at most 6 hours before Tₘᵢₙ, and when advancing the phase, light exposure needs to be administered at most 6 hours after Tₘᵢₙ.

Any experienced traveler can confirm that when travelling eastward, shifting of the internal clock to the new time is slower than when travelling westward. Acquaintance to the new daily rhythm is simply slower and more difficult when travelling eastward. When flying westward the daily rhythm in the destination time zone is behind the circadian rhythm of the traveler. However, when flying eastward the daily rhythm in the destination time zone is ahead of the circadian rhythm of the traveler. This can be exemplified:
A Finnish person living in Helsinki Finland travels to New York, United States of America which corresponds to crossing of 7 time zones. In Helsinki the travelers' Tₘᵢₙ is normally at 4 am, which corresponds to 21.00 (9 pm) in New York time. Thus, when he arrives in New York his internal circadian rhythm is ahead of the local New York rhythm. To align his internal circadian rhythm and the local New York daily rhythm he needs to delay his phase which corresponds to delaying Tₘᵢₙ. His Tₘᵢₙ will occur at 21.00 and taking one or more doses of bright light between 15.00 and 21.00 will delay Tₘᵢₙ and move the phase forward.

An American living in New York travels to Helsinki which again corresponds to crossing of 7 time zones. The Americans' Tₘᵢₙ is normally at 4 am in New York, which corresponds to 11 am Helsinki time. Thus, when he arrives in Helsinki his internal circadian rhythm is behind the local Helsinki rhythm. If he arrives during the forenoon in Helsinki it will be thus be coincident with his Tₘᵢₙ and his mental and cognitive performance will typically be at a minimum. To align his internal circadian rhythm and the local Helsinki daily rhythm he needs to advance his phase which corresponds to move Tₘᵢₙ backward in time, i.e. he needs to move Tₘᵢₙ from 11 am and back to 4 am. As stated above he thus needs to wait until after Tₘᵢₙ, i.e. after 11 am, before Tₘᵢₙ can be moved back in time.

A further challenge when travelling east is that the phase shift that can be achieved with bright light is larger when delaying Tₘᵢₙ than when advancing Tₘᵢₙ. It is estimated that the phase shift that can be achieved when delaying Tₘᵢₙ is 2-3 hours whereas the phase shift that can be achieved when advancing Tₘᵢₙ is only 1-1.5 hours. Thus, if the American from the example above tries to advance his Tₘᵢₙ by means of bright light therapy, Tₘᵢₙ will only be moved from 11 am to around 9.30-10 am, which is still completely out of sync with the local Helsinki time.

A further embodiment of the invention therefore relates to system for preventing symptoms of jetlag of a user travelling across time zones, said user having a time of daily temperature minimum Tₘᵢₙ, said system comprising
- one or more radiation units adapted to direct bright light at one or more neuroanatomical brain structures of the individual from at least one extra-cranial non-ocular position, and
- planning means for calculating a westbound dose scheme for travelling west and an eastbound dose scheme for travelling east, said dose schemes adapted to be applied to the user at the day of arrival, wherein
   - the westbound dose scheme comprises one or more doses of bright light scheduled up until 6 hours before Tₘᵢₙ and adapted to increase alertness of the user, and one or more doses of bright light scheduled within 4 hours before Tₘᵢₙ and adapted to delay Tₘᵢₙ of the user, and
   - the eastbound dose scheme comprises:
      - when crossing less than 6 time zones: one dose of bright light scheduled between 1 and 4 hours after Tₘᵢₙ, said dose adapted to advance Tₘᵢₙ of the user, and a plurality of doses of bright light scheduled more than 6 hours after Tₘᵢₙ, each of said doses adapted to increase alertness of the user, and
      - when crossing at least 6 time zones: a plurality of doses of bright light scheduled between 1 and 4 hours before Tₘᵢₙ, each of said doses adapted to delay Tₘᵢₙ of the user.

The present invention is at least partly based on the finding that instead of moving Tₘᵢₙ backwards in time (i.e. advancing Tₘᵢₙ) for alleviating symptoms of jetlag after long distance eastbound travelling, Tₘᵢₙ can be moved continuously forward during the day of arrival. The main advantage is that advancement of Tₘᵢₙ can only be achieved only once per day whereas delaying of Tₘᵢₙ can be obtained several times per day because each dose of bright light applied up to 6 hours before Tₘᵢₙ can move Tₘᵢₙ 2-3 hours forwardly.

A limit of crossing of 6 time zones is stated above. The present inventors have estimated that when crossing about 6 time zones or more Tₘᵢₙ is advantageously moved forward during the day of arrival, whereas when crossing less than 6 time zones Tₘᵢₙ is advantageously moved backward in time. However, please note that this is an approximate limit and in further embodiments of the invention this limit may be 4, 5, 7 or 8 time zones.

The present invention is also at least partly based on the observation that in addition to phase shifting efficacy, light therapy also increases alertness and performance. Thus, the doses planned outside the ±6 hour time window of Tₘᵢₙ are primarily prescribed to increase the alertness of the user, whereas the doses planned within the ±6 hour time window of Tₘᵢₙ are primarily prescribed to delay or advance Tₘᵢₙ; however it should not be neglected that these entraining doses may also have an positive effect on the alertness of the user.

### Description of Drawings

Fig 1. Phase response curve of light and melatonin. The triangle indicates the time of daily temperature minimum Tₘᵢₙ (approx. 4 am).
Fig. 2. Illustration of the diurnal rhythms. The top curve shows the core temperature (dashed line) whereas the bottom curves show simple and complex performance tasks (full lines). The dotted line shows the time-course of a complex performance test that would occur in the absence of effects due to fatigue.
Fig. 3. An example of a westbound dose scheme with the number of crossed time zones indicated above the scheme. The dose scheme comprises four daily doses of bright light separated by intervals of three hours and distributed over four days beginning at the day of arrival. The times are indicated in the destination time zone.
Fig. 4. An example of an eastbound dose scheme with the number of crossed time zones indicated above the scheme. The dose scheme comprises four daily doses of bright light separated by intervals of three hours and distributed over four days beginning at the day of arrival. The times are indicated in the destination time zone.
Figs. 5A-L. Eastbound day 1 (i.e. day of arrival) dose schemes illustrated as "jetlag wheels". Fig. 5A corresponds to 1 crossed time zone, fig. 5B corresponds to 2 crossed time zones etc., i.e. fig. 5L corresponds to 12 crossed time zones.
Figs. 6A-L. Eastbound day 2 (i.e. the day after the day of arrival) dose schemes illustrated as "jetlag wheels". Fig. 6A corresponds to 1 crossed time zone, fig. 6B corresponds to 2 crossed time zones etc., i.e. fig. 6L corresponds to 12 crossed time zones.
Figs. 7A-J: Westbound day 1 dose schemes illustrated as "jetlag wheels". Fig. 7A corresponds to 3 crossed time zones, fig. 7B corresponds to 4 crossed time zones etc., i.e. fig. 7J corresponds to 12 crossed time zones.
Figs. 8B-J: Westbound day 2 dose schemes illustrated as "jetlag wheels". Fig. 8B corresponds to 4 crossed time zones, fig. 8C corresponds to 5 crossed time zones etc., i.e. fig. 8J corresponds to 12 crossed time zones.
Fig. 9: Diagrammatic illustration of one embodiment of the present invention incorporated in a server system accessible via a communication network.

### Detailed description of the invention

It is widely believed that the effect of bright light on human psychophysiology is entirely mediated via the eyes. Based on earlier studies, however, it is known that several species - including mammals - significant amount of ambient light penetrates the skull and the brain (Ganong et al. 1963). In recent years knowledge of photosensitive proteins - opsins - in the brain has advanced. Opsins are transmembranic proteins that, when exposed to light, modify their conformity, and initiate a cascade (phototransduction) mediated by G-protein. This cascade is the basis for neural responses to light in target tissues containing opsins, such as the retina. Of all mammals at least rodents (Blackshaw et al. 1999, Lein et al. 2007) and humans (Allen Institute for Brain Science) have been long known to have opsin-genes in their genome in wide areas of the brain at transmitter-RNA-level. The present inventors have succeeded in proving the presence of photosensitive opsin proteins (OPN3, OPN4) widely at protein level in the brain of both men and mice, especially in the areas central to the control of bodily homeostasis and hormonal control, such as hypophysis, hypothalamus, pituitary gland and medulla oblongata, as well as substantia nigra and locus caeroleus central to the production and storage of dopamine and serotonin, respectively. (Nissilä et al. 2011, Nissilä et al. 2012). Transcranial light therapy has been shown to modulate in particular the motoric and visual cortex functions, but also causing significant activity change in s. nigra and locus caeroleus (Starck et al. 2012). Broad presence of opsins in several parts of the human brain, and the results of an fMRI study support the assumption that the brain is sensitive to transcranial light.

The bodily circadian rhythm is managed by the suprachiasmatic nuclei of the hypothalamus (SCN, internal clock). The "clock-genes" of the SCN cells produce "clock-proteins" that, among other things, inhibit the clock-gene (negative feedback). As the protein saturation decreases, the inhibition of the clock-genes, too, decreases and the clock-genes start their protein synthesis again (Takahashi et al. 2008). Typically a cycle lasts 24-25 hours (Clayton et al. 2001). The melatonin receptors (type 2) of the SCN cells are sensitive to the melatonin released by pineal gland. SCN also receives information of external light through the retinohypothalamic route of the eye (Dupocovich & Markowska 2005). IGL (intergeniculate leaflet) is also believed to transmit information about e.g. physical activity to the internal clock (Waterhouse 2007). The environmental light-dark cycle and melatonin are the most significant influencers of circadian rhythm (Zeigeber). In addition other factors such as sleeping, physical activity, and eating can influence the regulation of circadian rhythm (Shanahan & Czeisler 2000). These other factors are, however, much less significant (Reilly et al. 2009).

Melatonin and cortisol secretion has been shown to follow the external circadian rhythm (Arendt 1995). The pinealocytes of the pineal gland are mainly responsible for production of physiologically significant melatonin. In addition melatonin is produced in the intestine, bone marrow and skin. The internal clock regulates production of melatonin in the pineal gland utilizing the sympathetic nervous system (Reilly et al. 2009). Light is the primary environmental factor regulating secretion of melatonin. Exposure to light suppresses melatonin secretion, and so melatonin production varies with the light-dark cycle. Melatonin secretion in humans is at its greatest at midnight (2-4 am). During daytime melatonin level is very low. The ability of light to suppress melatonin secretion has been noted even at very low light intensity (Zawilska et al. 2009). Short wave length light (blue-green light) has been shown to block melatonin secretion and promote shifting of circadian rhythm better than long wave length light (Wright & Lack 2001). The ability of light to block melatonin secretion also depends on the time of day when light is introduced, the preceding ambient light level, and the duration of light exposure (Zawilska et al. 2009). Crossing of several time zones also causes a disturbance in the circadian rhythm of cortisol. Jetlag-experiences are possibly responsible for physical and psychological stress, and thereby increasing total daily secretion of cortisol (Cho et al. 1999). Chronically high level of cortisol has been noted to reduce cognitive performance (McEwan & Sapolsky 1995).

In one embodiment of the invention any two doses of bright light are separated by an interval of at least 1, 1.5, 2, 2.5, 3, 3.5, 4, 4.5 or 5 hours. Furthermore, the dose schemes may be scheduled to be prescribed during the daytime in the arrival time zone. The number of daily doses may be 1, 2, 3, 4 or at least 5. Support of having multiple doses per day with more than 2 hours interval between doses is provided by Hastings et al. (1999) wherein analysis of c-fos induction and cAMP response element-binding protein phosphorylation in the retinorecipient SCN demonstrated that the SCN are able to resolve and respond to light pulses presented 1 or 2 hours apart. Analysis of the phase shifts of the circadian wheel-running activity rhythm of hamsters presented with single or double pulses demonstrated that resetting of the oscillator occurred within 2 hour. This was the case for both delaying and advancing phase shifts. Examination of delaying shifts in the mouse showed resetting within 2 hours and in addition showed that resetting is not completed within 1 hour of a light pulse.

As stated previously there is a time window of 6 hours before Tₘᵢₙ to apply the doses when delaying Tₘᵢₙ. In one embodiment of the invention the eastbound dose scheme therefore comprises 4 doses of bright light scheduled at 4, 3, 2 and 1 hours, respectively, before Tₘᵢₙ. The first dose applied 4 hours before Tₘᵢₙ possibly moves Tₘᵢₙ 2 hours forward. If the next (second) dose is applied 3 hours later it will then be 3 hours before the new Tₘᵢₙ. This second dose will move the new Tₘᵢₙ two hours forward. The third dose applied 3 hours later is then 2 hours before the new Tₘᵢₙ and will again move Tₘᵢₙ. The fourth dose applied 3 hours later is then 1 hour before the corrected Tₘᵢₙ and will again move Tₘᵢₙ. At the end of the day Tₘᵢₙ has been pushed 8 hours forward (2 hours per dose). Following the example above with the American flying to Helsinki, his Tₘᵢₙ has been moved from 11 am to 19 (7 pm). The four doses applied through the day have pushed Tₘᵢₙ in front of him thereby avoiding the decrease in cognitive performance which is correlated with Tₘᵢₙ. His alertness may further have been lifted by the doses of bright light applied throughout the day.

As Tₘᵢₙ can be moved 2-3 hours forward per dose, the doses may be scheduled to constantly be 2-3 hours before Tₘᵢₙ with intervals of 2-3 hours, e.g. 4 doses of bright light scheduled 2 hours before Tₘᵢₙ with 2 hours intervals or 4 doses of bright light scheduled 3 hours before Tₘᵢₙ with 3 hours intervals.

In one embodiment of the invention the bright light is administered through the auditory canals of the user. This is for example the case if using the Valkee brain stimulation headset. Furthermore, a dose of bright light may correspond to 8-12 minutes of bright light, such as 1, 2, 3, 4, 5, 6, 7, 8, 9, 10, 11, 12, 13, 14 or 15 minutes of bright light. The luminous flux in each ear in a dose of bright light may correspond to 0.5-15 lumens, such at least 0.2, 0.5, 1, 2, 3, 4, 5, 6, 7, 8, 9, 10, 11, 12 lumens and such as less than 15, 14, 13, 12, 11, 10, 9, 8, 7, 6, 5, 4, 3, 2 lumens or less than 1 lumen.

The time of daily temperature minimum Tₘᵢₙ may be obtained as user input. The travelling user may know his personal Tₘᵢₙ or he may be asked to indicate the time of waking up in the morning. Tₘᵢₙ can then be found by subtracting 2-3 hours. In a further embodiment Tₘᵢₙ of the user is predefined, such as predefined to be at 3.00, 3.30, 4.00, 4.30 or 5.00 o'clock in the departure time zone. E.g. predefining Tₘᵢₙ to be at 4 am would apply to most people.

The day of arrival is the most important day to start applying the bright light treatment. However, one day may not be enough to fully synchronize the circadian rhythm with the new daily rhythm at the destination. Thus, in a further embodiment of the invention the westbound dose scheme and/or the eastbound dose scheme further comprises 1-4 doses of bright light scheduled at the day after the day of arrival. Furthermore, the westbound dose scheme and/or the eastbound dose scheme may comprise 1-4 doses of bright light scheduled two days after the day of arrival. And even further the westbound dose scheme and/or the eastbound dose scheme may comprise 1-4 doses of bright light scheduled three days after the day of arrival.

In one embodiment of the invention and when travelling west the time of the first dose at the second day after arrival is scheduled between 8 and 10 o'clock, such as 9 o'clock, if crossing less than seven time zones, and between 11 and 13 o'clock, such as 12 o'clock, if crossing at least seven time zones. Furthermore, when travelling west the time of the first dose at the third day after arrival may be scheduled between 8 and 10 o'clock, such as 9 o'clock. And further, when travelling west the time of the first dose at the fourth day after arrival may be scheduled between 8 and 10 o'clock, such as 9 o'clock.

In one embodiment of the invention and when travelling east, the time of the first dose at the second day after arrival is scheduled between 8 and 11 o'clock, such as 9 or 10 o'clock. Furthermore, when travelling east the time of the first dose at the third day after arrival may be scheduled between 8 and 10 o'clock, such as 9 o'clock. And further, when travelling east the time of the first dose at the fourth day after arrival may be scheduled between 8 and 10 o'clock, such as 9 o'clock.

A further embodiment of the invention relates to a computer program product having a computer readable medium, said computer program product suitable for calculating a schedule of bright light treatments for preventing symptoms of jetlag when crossing a number of time zones during travelling, said computer program product comprising means for carrying out all the steps of the method as herein defined.

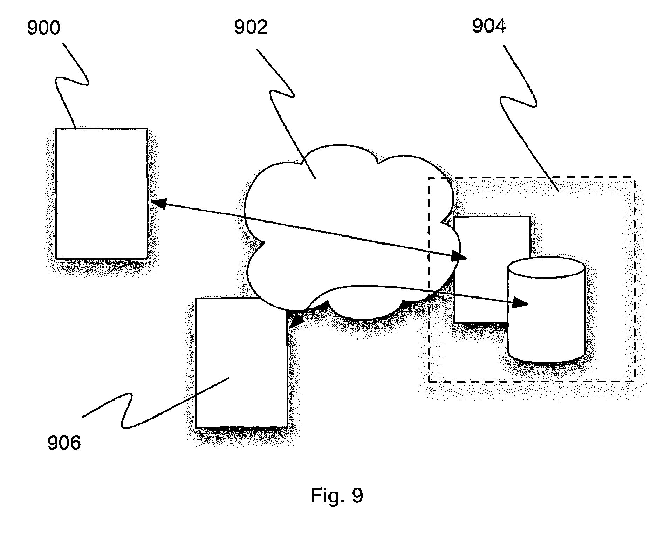

According to a further embodiment of the invention the computer program can be run in a mobile terminal, smart phone, laptop or dedicated light therapy device etc. The program can be standalone software (in the form of an application) or it can use on-line information. Preferably the computer program for controlling jetlag dosing runs in a light therapy device such as the Valkee light therapy device. Additionally the program can run in server system 904 accessible via communication network 902 such as the Internet (see fig. 9). The server system 904 can have the entire functionality or it can provide schedule data for the application running in a device 900 in which the computer program runs. The server system 904 can include features of collecting feedback from the users. Said feedback can be used to further modify parameters of the light therapy if needed. Feedback can be sent to the server system 904 directly from the device 900 or it can be given for example by using a web-interface with a web browser. The server system 904 can be configured to share information and data to third party services 906 such as Facebook / Twitter etc. The server system 904 can be configured to receive information from other third party systems such as a travel management system (like Amadeus) to receive traveller data for proposing/programming jetlag schedule. The server system 904 can be configured to send data, proposals, software updates, updates on parameters etc. to the device 900 over the communication network 902.

The dose schemes as provided by the present invention can be seen as an advice on how to avoid symptoms of jetlag. It is off course voluntary for the user to apply the doses as advised. The time of the doses can also be seen as recommended approximate times and it is not crucial for the user to take a dose at exactly the time that is prescribed by a dose scheme. E.g. a dose scheduled at 11 o'clock can be applied between approx. 10.45 and 11.15 or even between 10.30 and 11.30.

### Examples

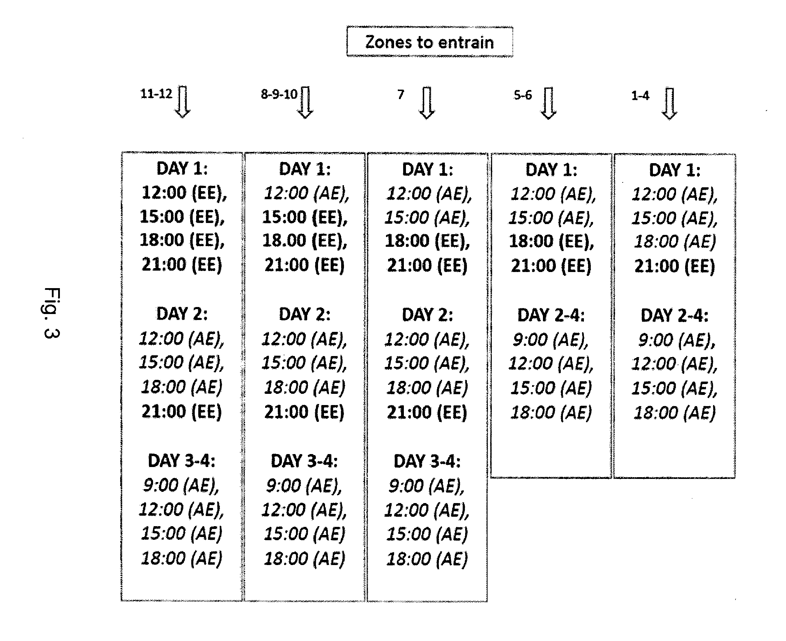

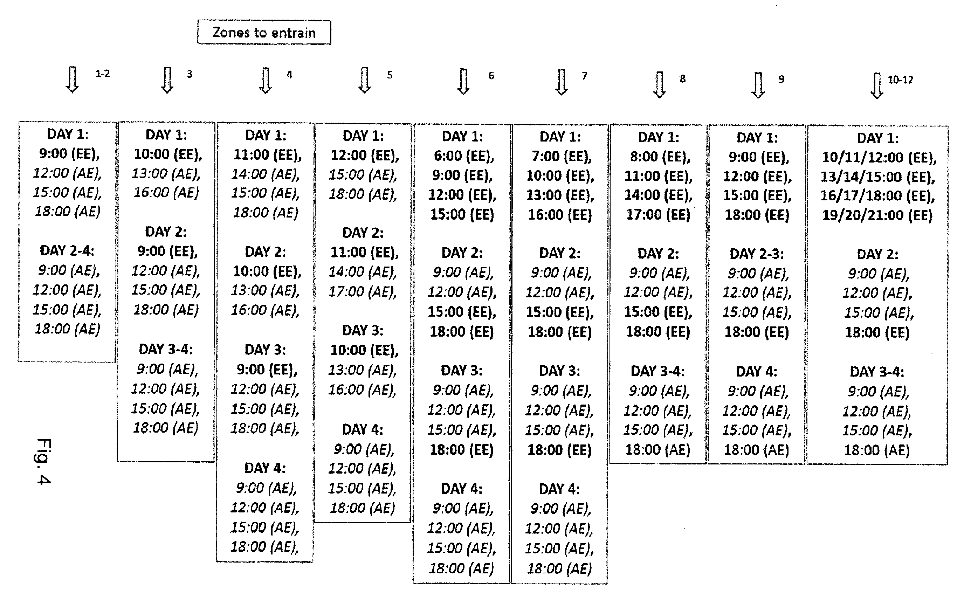

Examples of westbound and eastbound dose schemes are provided in figs. 3 and 4. Fig. 3 shows an example of a westbound dose scheme with the number of crossed time zones indicated above the scheme. The dose scheme comprises three to four daily doses of bright light separated by intervals of three hours and distributed over four days beginning at the day of arrival. The times are indicated in the destination time zone. After each dose is written "(AE)" for "Acute effect" or "(EE)" for entraining effect indicating what the specific dose is adapted for. It can be seen that at day 1 the dose scheme begins at 12.00 no matter how many time zones to be crossed. The difference is in the effect of each dose. When crossing 1-4 time zones the first three doses are adapted to increase alertness of the user, i.e. an acute effect, whereas the last dose at 21.00 (9 pm) is within the 6 hour time window of Tₘᵢₙ and has the effect that Tₘᵢₙ is delayed, i.e. an entraining effect. When 5-6 time zones are crossed the dose scheduled at 18.00 also has an entraining effect and when 11-12 time zones are crossed (or more) every dose is adapted to delay Tₘᵢₙ. On day 2 the circadian rhythm is still not properly adjusted when 7 time zones or more are crossed and the dose scheme therefore begins at 12 and ends at 21 with only the last dose having an entraining effect. With less than 7 time zones crossed the dose scheme begins at 9 and the bright light doses are primarily schedule to have an effect on the alertness of the user. On days 3 and 4 all dose schemes begin at 9 am.

Fig. 4 shows an example of an eastbound dose scheme with the number of crossed time zones indicated above the scheme. The dose scheme comprises four daily doses of bright light separated by intervals of three hours and distributed over four days beginning at the day of arrival. The times are indicated in the destination time zone. It can be seen than when less than 6 time zones are crossed the first dose is planned 3 hours after Tₘᵢₙ (which is predefined to be at 4 am in the departure time zone). On day 2 the first dose is moved 1 hour backward to account for the 1 hour advancement of Tₘᵢₙ that took place on day 1. On day 3 the first dose is again moved 1 hour backward to account for the additional 1 hour advancement of Tₘᵢₙ that took place on day 2, and so on. However, it can also be seen that the first dose is never scheduled before 9 in the morning. When crossing less than 6 time zones only the first dose has an entraining effect of advancing Tₘᵢₙ, whereas the remaining doses are outside the 6 hour time window of Tₘᵢₙ and are adapted to increase alertness of the user.

When crossing at least 6 time zones the first dose is planned earlier and is thereby scheduled before Tₘᵢₙ to be able to delay Tₘᵢₙ. Each dose of the first day is adapted to delay Tₘᵢₙ and thereby continuously push Tₘᵢₙ in front of the user. On day 2 the dose schemes are identical whether crossing 6 or 12 times zones, however when crossing 6 time zones the last two doses are adapted to delay Tₘᵢₙ.

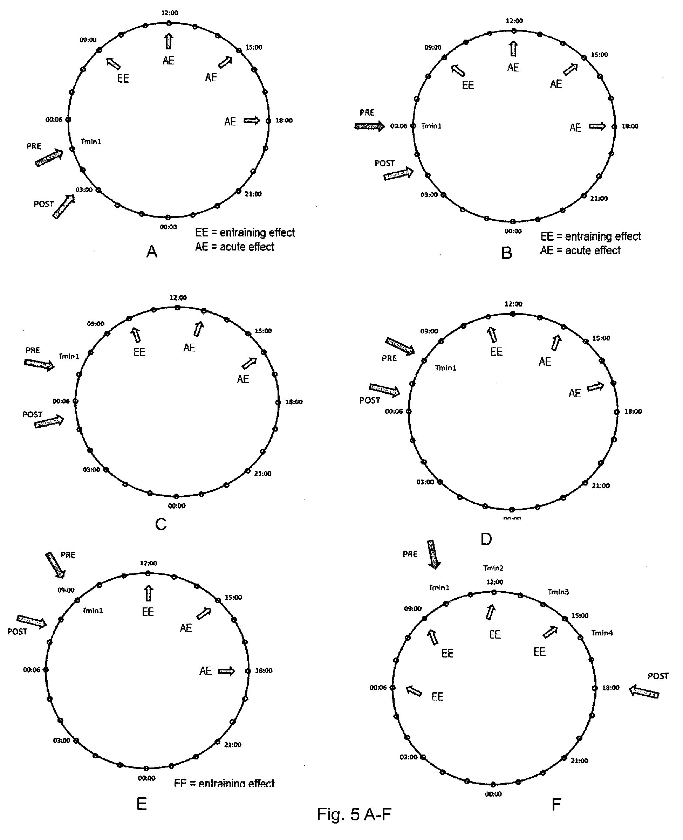

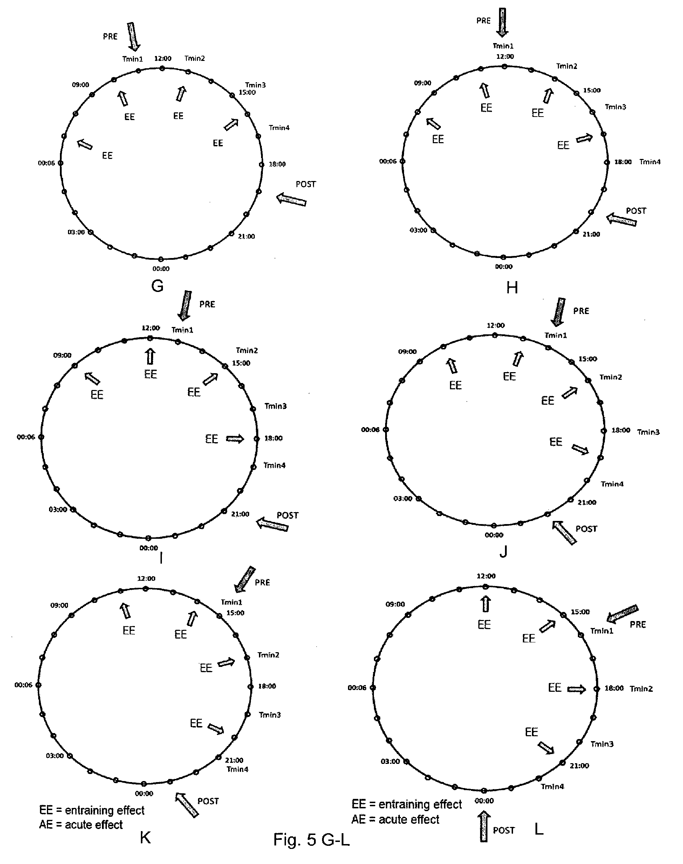

Figs. 5-8 show another way of illustrating dose schemes in the form of "jetlag wheels". This jetlag wheel may be a way of illustrating the specific dose scheme for a user that has entered his travel plans. Fig. 5A is a day one jetlag wheel for crossing 1 time zone. The arrows outside the wheel indicate the time of Tₘᵢₙ before ("PRE") and after ("POST") light exposure. In this case Tₘᵢₙ is predefined to be at 4 o'clock indicated by the "pre" arrow pointing at 5 o'clock (4 o'clock plus 1 hour time difference). The time of the doses are indicated by the arrows inside the wheel with the first EE arrow pointing at 9 o'clock. This dose is 4 hours after Tₘᵢₙ, i.e. within the 6 hour time window and has the entraining effect of advancing Tₘᵢₙ 1.5 hours to 3.30 indicated by the POST arrow pointing at 3.30. The three AE arrows at 12, 15 and 18 indicate the time of the remaining doses having an acute effect, i.e. increasing alertness of the user. The same principle continues in fig. 5B (day one, 2 time zones east), 5C (day one, 3 time zones east), 5D (day one, 4 time zones east) and 5E (day one, 5 time zones east), however with only three daily doses in fig. 5C-E.

Fig. 5F is a day one eastbound jetlag wheel for crossing 6 time zones. Tₘᵢₙ is now continuously delayed through the day beginning at 10 o'clock indicated by the PRE arrow and by T_{min,1} and ending at the POST arrow at 18 o'clock after being delayed four times by the EE doses. It can be seen from fig. 5F that the doses are separated by three hours and each dose delays Tₘᵢₙ with 2 hours (T_{min,1} →T_{min,2} →T_{min,3} →T_{min,4}). The same principle applies in figs. 5G-L with the jetlag wheel being shifted 1 hour each time.

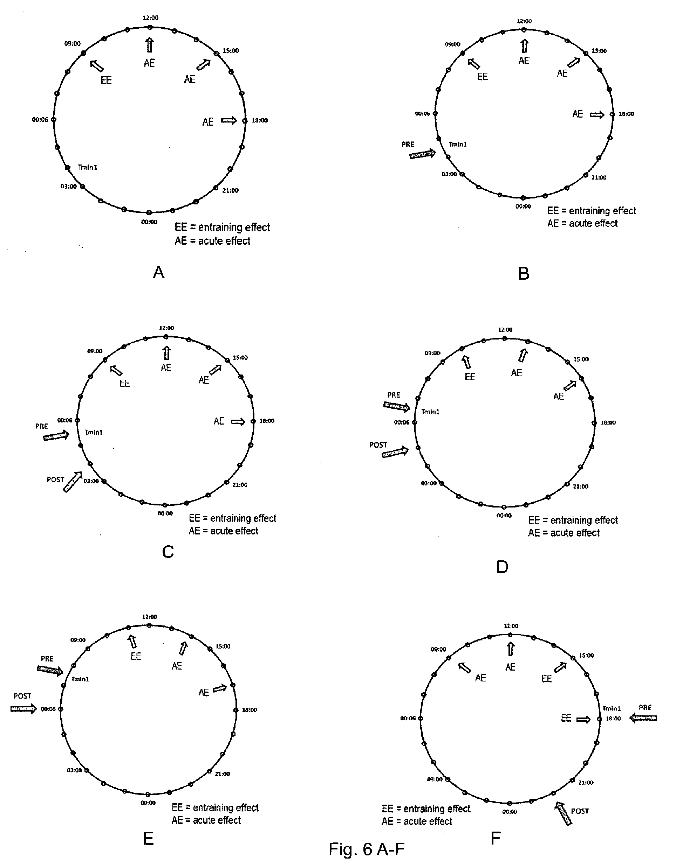

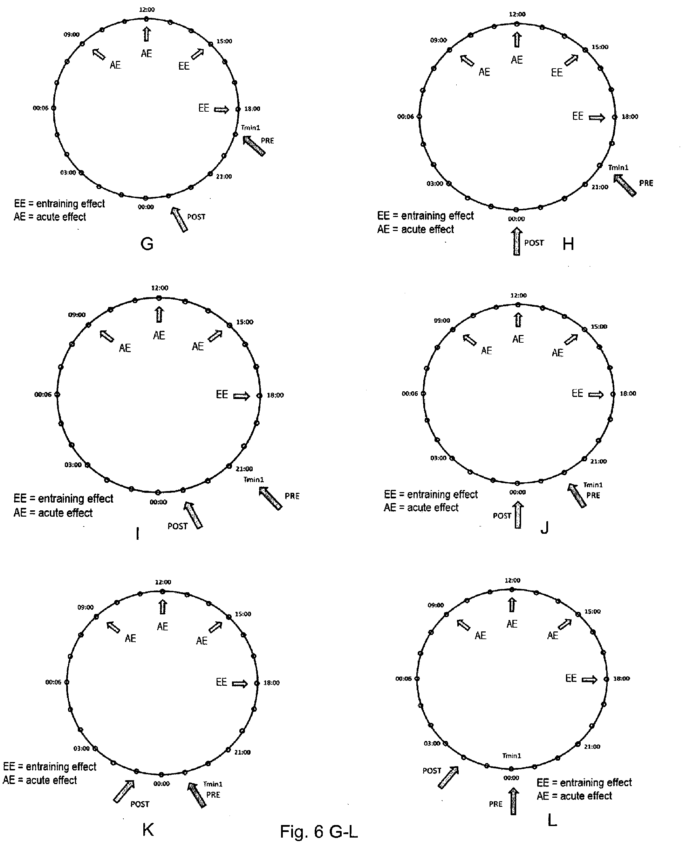

Figs. 6A-L shows day two eastbound jetlag wheels for crossing one (fig. 6A) to twelve (fig. 6L) time zones.

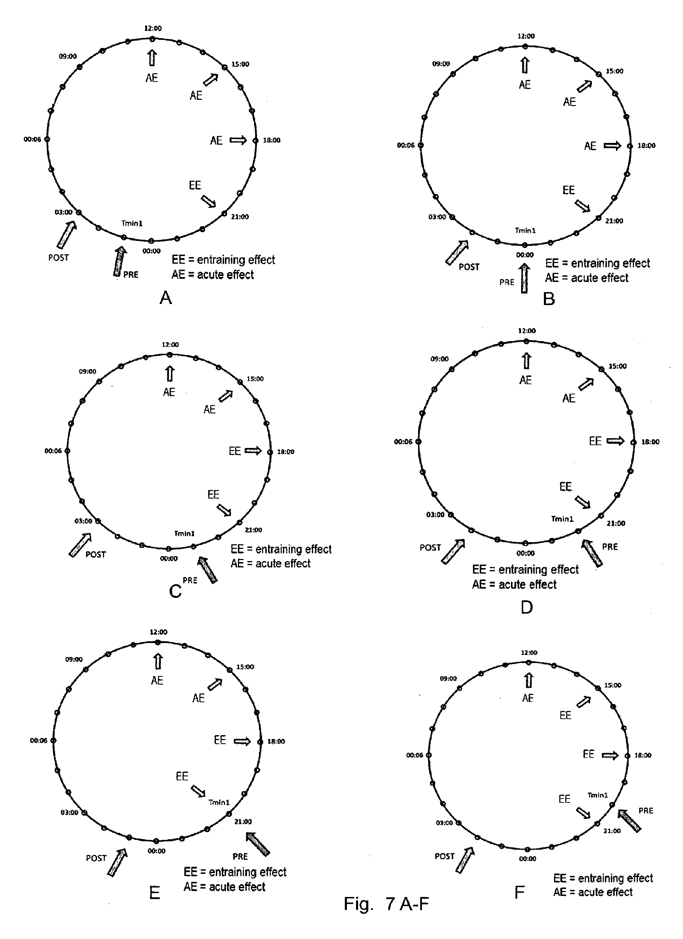

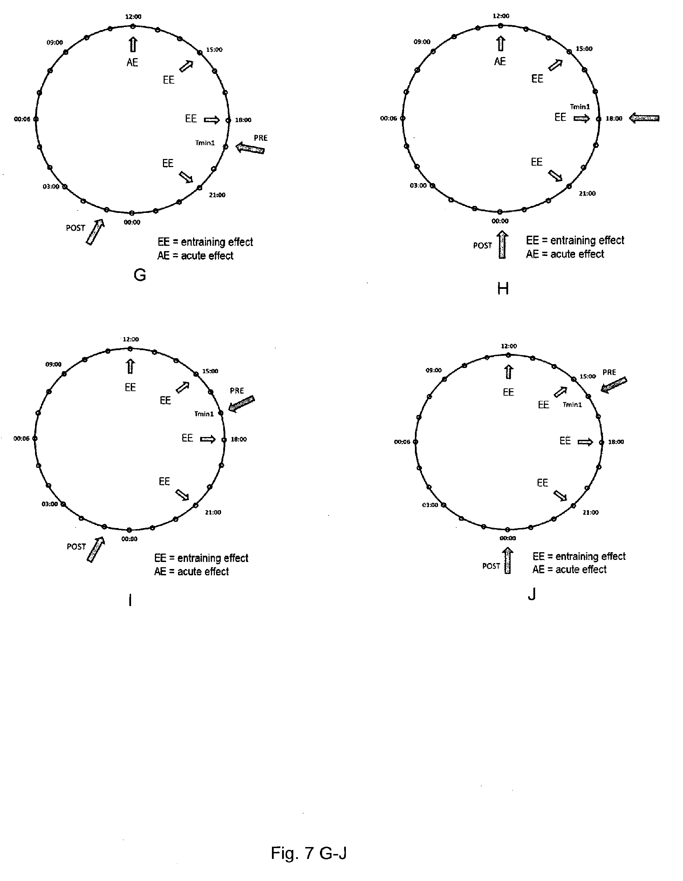

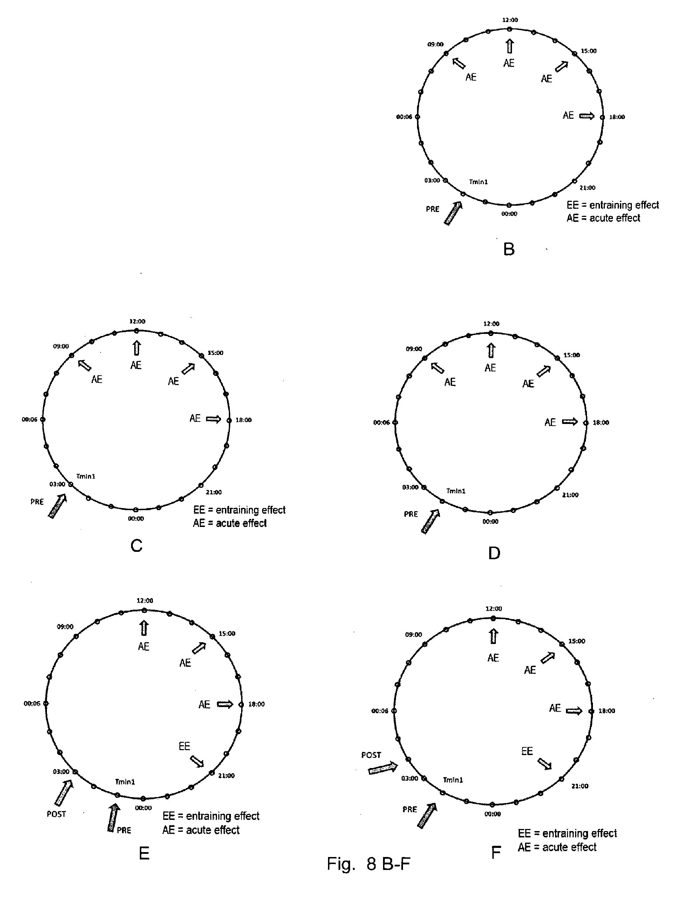

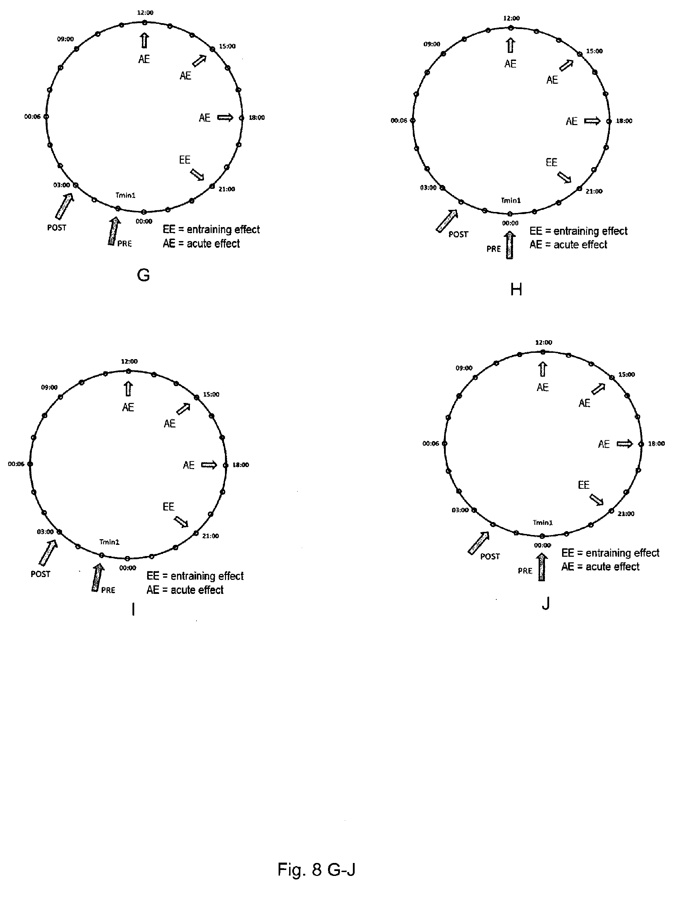

Fig. 7A-J shows day one westbound jetlag wheels for crossing three (fig. 7A) to twelve (fig. 7J) time zones and fig. 8B-J shows day two westbound jetlag wheels for crossing four (fig. 8B) to twelve (fig. 8J) time zones. The principles as described above for the eastbound jetlag wheels are the same.

### Additional usage scenario

A team (11) of top athletics were using a similar device as described in WO 2008/029001 wherein bright LED light is provided to the brain through the auditory canal. During the experiment the team travelled seven time zones to the east and stayed there for seven days before returning back to home. The team used the device to assist with acclimatisation. The device was used for entrainment of the circadian rhythm with the intention to stay in the departure time zone during the travel. The Valkee light device was used as a substitute for times when the team should have had light exposure according to the departure timescale, but there was no ambient light outside in the destination time zone. In effect an "own" time zone was created that would assist with resumption of normal biorhythms when returning to the departure time zone.

The test persons took light therapy sessions (i.e. doses of bright light) when waking up in the morning followed by natural light exposure and then three hourly top-ups once darkness had fallen to a maximum of four doses per day.

When back from east the device was advised to be used at 11 am for the following three days with the additional three hourly top-ups.

Based on the conducted experiment it was found out that the test persons were able to maintain the departure time zone at the destination for seven days and they did not suffer from jetlag due to the entrainment. Their sleeping pattern was followed during and after the trip. No significant disturbance in sleeping hours and patterns were observed. In addition the subjective feedback from the test persons indicated no issues with jetlag.

### References

Arendt J. Melatonin and the mammalian pineal gland. London: Chapman Hall 1995.
Auger RR & Morgenthaler TI. Jet lag and other sleep disorders relevant to the traveller. Travel medicine and infectious disease 2009;7:60-68.
Blackshaw, S. & Snyder, S.H. 1999. Encephalopsin: a novel mammalian extraretinal opsin discretely localized in the brain. J. Neurosci. 19, 3681-3690
Burgess HJ, Crowley SJ, Gazda CJ, Fogg LF, Eastlam CI. Preflight adjustment to eastward travel: 3 days of advancing sleep with and without morning bright light. J Biol Rhythms 2003;18:318-28.
Cajochen C. Alerting effects of light. Sleep Medicine reviews. 2007;11:453-464.
Campbell SS, Murphy PJ, Suhner AG. Extraocular phototransduction and circadian timing systems in vertebrates. Chronobiol Int 2001;18:137-72.
Cho K, Ennaceur A, Cole JC, Suh CK. Chronic jet lag procedures cognitive deficit. J Neurosci 2000;20:1-5.
Clayton J, Kyriacou C, Reppert S. Keeping time with the human genome. Nature 2001;409:829-31.
Eastman CI, Gazda CJ, Burgess HJ, Crowley SJ, Fogg LF.Advancing circadian rhythms before eastward flight: a strategy to preventor reduce jetlag. Sleep 2005:28;33-44.
Ganong WF, Shepherd MD, Wall JR, Van Brunt EE, Clegg MT. Penetration of light into the brain of mammals. Endocrinology. 1963;72:962-3.
Gooley JJ. Treatment of circadian rhythm sleep disorders with light. Ann Acad Med Singapore. 2008;37:669-76.
Haimov I, Arendt J. The prevention and treatment of jetlag. Sleep Medicine reviews. 1999;3:229-40.
Hastings MH, Best JD, Maywood ES and Smith KL. Rapid Resetting of the Mammalian Circadian Clock. The Journal of Neuroscience, January 15, 1999, 19(2):828-835.
Jurvelin H, Nissilä J, Timonen M, Takala T, Jokelainen J, Räsänen P. Transcranial Bright Light Treatment via Ear Canals in Seasonal Affective Disorder (SAD) - a Randomized Controlled Trial. International Journal of Psychiatry in Clinical Practice, 2011: 15 (Suppl 2): 27-28.
Khalsa SS, Jewett ME, Cajochen J, CzeislerCA. A phase response curveto single bright light pulses in human subjects. J Physiol. 2003;549:945-52.
Lein, E.S., Hawrylycz, M.J., Ao, N., Ayres, M., Bensinger, A. et al. 2007. Genome-wide atlas of gene expression in the adult mouse brain.Genome-wide atlas of gene expression in the adult mouse brain. Nature 445, 168-176.
McEwen BS & Sapolsky RM. Stress and cognitive function. Curr Opin Neurobiol 1995;5:205-216.
Nissilä J, Mänttäri S, Tuominen H, Särkioja T, Timonen M, Saarela S. The Abundance and Distribution of Encephalopsin (OPN3) Protein in Human Brain. Abstract for the Scandinavian Physiological Society's Annual Meeting, 12-14 August. 2011, Bergen Norway. Ss 107.
Nissilä J, Mänttäri S, Tuominen H, Särkioja T, Takala T, Timonen M, Saarela S. The abundance and distribution of melanopsin (opn4) protein in the human brain. Abstract for the EPA annual meeting 3-6 March. 2012, Prague, Czech Republic.
Recht LD, Lew RA, Schwartz WJ. Baseball teams beaten by jetlag. Nature 1995;377:583.
Reilly T, Waterhouse J, Edwards B. Some chronobiological and physiological problems associated with long-distance journeys. Travel Med Infect Dis 2009;7:88-101.
Reilly T, Waterhouse J, Edwards B. Jet lag and air travel: Implication for performance. Clin Sport Med 2005;24:367-80.
Revell VL, Eastman CI. How to trick mother nature into letting you fly around and stay up all night. J Biol Rhythms. 2005;20:353-65.
Takahashi JS, Hong HK, Ko CH, McDearmon EL. The genetics of mammalian circadian order and disorder: Implication for physiology and disease. Nat Rev Genet 2008;9:764-775.
Timonen M, Jokelainen J, Jurvelin H, Nissilä J, Aunio A, Räsänen P, Takala T. Can transcranial brain-targeted bright light treatment via ear canals be effective in relieving symptoms in seasonal affective disorder? A pilot study. Med Hypotheses. 2012;78:511-5.
Van Brunt EE, Shephard MD, Wall JR, Ganong WF, Clegg MT. Penetration of light into the brain of mammals. Ann N Y Acad Sci 1964;117:217-24.
Waterhouse J, Reilly T, Atkinson G, Edwards B. Jetlag: trends and coping strategies. Lancet 2007;369:1117-29.
Winget CM, DeRoshia CW, Markley CL, Holley DC. A review of human physiological and performance changes associated with desynchronosis of biological rhythms. Aviat Space Environ Med. 1984;55:1085-1096.
Wright H & Lack L. Effect of light wavelength on suppression and phase delay of the melatonin rhythm. Chronobiol Int 2001;18:801-08.
Zawilska JB, Skene DJ, Arendt J. Physiology and pharmacology of melatonin in relation to biological rhythms. Pharmacological Reports. 2009;61:383-410.

## Claims

1. A system for preventing symptoms of jetlag of a user travelling across time zones, said user having a time of daily temperature minimum Tₘᵢₙ, said system comprising
- one or more radiation units adapted to direct bright light at one or more neuroanatomical brain structures of the individual from at least one extra-cranial non-ocular position, and
- planning means for calculating a westbound dose scheme for travelling west and an eastbound dose scheme for travelling east, said dose schemes adapted to be applied to the user at the day of arrival, wherein
- the westbound dose scheme comprises one or more doses of bright light scheduled up until 6 hours before Tₘᵢₙ and adapted to increase alertness of the user, and one or more doses of bright light scheduled within 4 hours before Tₘᵢₙ and adapted to delay Tₘᵢₙ of the user, and
- the eastbound dose scheme comprises:
• when crossing less than 6 time zones: one dose of bright light scheduled between 1 and 4 hours after Tₘᵢₙ, said dose adapted to advance Tₘᵢₙ of the user, and a plurality of doses of bright light scheduled more than 6 hours after Tₘᵢₙ, each of said doses adapted to increase alertness of the user, and
• when crossing at least 6 time zones: a plurality of doses of bright light scheduled between 1 and 4 hours before Tₘᵢₙ, each of said doses adapted to delay Tₘᵢₙ of the user.

2. The system according to any of the preceding claims, wherein any two doses of bright light are separated by at least 2 hours or at least 3 hours.

3. The system according to any of the preceding claims, wherein the eastbound dose scheme comprises 4 doses of bright light scheduled at 4, 3, 2 and 1 hours, respectively, before Tₘᵢₙ.

4. The system according to any of the preceding claims, wherein the eastbound dose scheme comprises 4 doses of bright light scheduled at 2 hours before Tmin.

5. The system according to any of preceding claims, wherein the bright light is administered through the auditory canals of the user, and/or wherein a dose of bright light corresponds to 8-12 minutes of bright light.

6. The system according to any of preceding claims, wherein Tₘᵢₙ is obtained as user input or wherein Tₘᵢₙ of the user is predefined, such as predefined to be at 3.00, 3.30, 4.00, 4.30 or 5.00 o'clock in the departure time zone.

7. The system according to any of preceding claims, wherein the westbound dose scheme and/or the eastbound dose scheme further comprises 1-4 doses of bright light scheduled at the day after the day of arrival.

8. The system according to any of preceding claims, wherein the westbound dose scheme and/or the eastbound dose scheme further comprises 1-4 doses of bright light scheduled two days and three days, respectively, after the day of arrival.

9. A computer implemented method for calculating a schedule of bright light treatments for preventing symptoms of jetlag when crossing a number of time zones during travelling, the method comprising calculating a schedule of doses of bright light beginning at the day of arrival, each dose separated by an interval of between 2 and 4 hours, such as 3 hours, wherein
- when travelling west the time of the first dose at the day of arrival is scheduled during the forenoon between 11 and 13 o'clock, such as 12 o'clock, and
- when travelling east the time of the first dose at the day of arrival is scheduled at around x+7 o'clock if crossing less than six time zones, and at around x o'clock if crossing at least six time zones, where x is the number of time zones crossed.

10. The method according to claim 9, wherein when travelling west the time of the first dose at the second day after arrival is scheduled between 8 and 10 o'clock, such as 9 o'clock, if crossing less than seven time zones, and between 11 and 13 o'clock, such as 12 o'clock, if crossing at least seven time zones.

11. The method according to any of claims 9 to 10, wherein when travelling west the time of the first dose at the third day after arrival is scheduled between 8 and 10 o'clock, such as 9 o'clock, and the time of the first dose at the fourth day after arrival is scheduled between 8 and 10 o'clock, such as 9 o'clock.

12. The method according to any of claims 9 to 11, wherein when travelling east the time of the first dose at the second day after arrival is scheduled between 8 and 11 o'clock, such as 9 or 10 o'clock, and the time of the first dose at the third day after arrival is scheduled between 8 and 10 o'clock, such as 9 o'clock.

13. The method according to any of claims 9 to 12, wherein when travelling east the time of the first dose at the fourth day after arrival is scheduled between 8 and 10 o'clock, such as 9 o'clock.

14. The method according to any of claims 9 to 13, wherein the number of daily doses is 3 or at least 4 and wherein a dose of bright light corresponds to 8-12 minutes of bright light administered through the auditory canals of a user.

15. A computer program product having a computer readable medium, said computer program product suitable for calculating a schedule of bright light treatments for preventing symptoms of jetlag when crossing a number of time zones during travelling, said computer program product comprising means for carrying out all the steps of the method as defined in any of claims 9 to 14, when executed on a data-processing system.

## Patentansprüche

1. System zum Verhindern von Jetlag-Symptomen eines Nutzers der über Zeitzonen reist, wobei der Nutzer eine Zeit täglichen Temperatunninimums Tₘᵢₙ hat, worin das System umfasst,
- ein oder mehrere Strahlungseinheiten, die angepasst sind, helles Licht auf eine oder mehrere neuroanatomische Gehirnstrukturen eines Individuums aus mindestens einer extrakranialen, nicht-okularen Position zu richten, und
- Planungsmittel zum Berechnen eines Westwärts-Dosisschemas zum Reisen nach Westen und eines Ostwärts-Dosisschemas zum Reisen nach Osten, worin das Dosisschema angepasst ist, dem Nutzer am Ankunftstag verabreicht zu werden, worin
- das Westwärts-Dosisschema umfasst: ein oder mehrere Dosen hellen Lichts, zu verabreichen bis 6 Stunden vor Tₘᵢₙ, und angepasst, die Wachsamkeit des Nutzers zu steigern, und ein oder mehrere Dosen hellen Lichts zu verabreichen innerhalb von 4 Stunden vor Tₘᵢₙ und angepasst, die Tₘᵢₙ des Nutzers zu verzögern, und
- das Ostwärts-Dosischema umfasst:
- beim Durchqueren von weniger als 6 Zeitzonen: eine Dosis hellen Lichts zwischen 1 und 4 Stunden nach Tₘᵢₙ, wobei die Dosis angepasst ist, die Tₘᵢₙ des Nutzers vorzuschieben, und mehrere Dosen hellen Lichts mehr als 6 Stunden nach Tₘᵢₙ, worin jede der Dosen angepasst ist, die Wachsamkeit des Nutzers zu erhöhen,
- beim Durchqueren von mindestens 6 Zeitzonen: mehrere Dosen hellen Lichts, zu verabreichen zwischen 1 und 4 Stunden vor Tₘᵢₙ, worin jede Dosis angepasst ist, die Tₘᵢₙ des Nutzers zu verzögern.

2. System nach einem der vorstehenden Ansprüche, worin jeweils 2 Dosen hellen Lichts um mindestens 2 Stunden oder mindestens 3 Stunden getrennt sind.

3. System nach einem der vorstehenden Ansprüche, worin das Ostwärts-Dosisschema 4 Dosen an hellem Licht umfasst, zu verabreichen jeweils 4, 3, 2 und 1 Stunden vor Tₘᵢₙ.

4. System nach einem der vorstehenden Ansprüche, worin das Ostwärts-Dosisschema 4 Dosen hellen Lichts umfasst, zu verabreichen 2 Stunden vor Tₘᵢₙ.

5. System nach einem der vorstehenden Ansprüche, worin das helle Licht über die Hörkanäle des Nutzers verabreicht wird und/oder worin eine Dosis hellem Lichts 8-12 Minuten hellem Licht entspricht.

6. System nach einem der vorstehenden Ansprüche, worin Tₘᵢₙ erhalten wird als Nutzer-Input oder worin die Tₘᵢₙ des Nutzers vorab definiert wird, so wie definiert bei 0300, 0330, 0400, 0430 oder 0500 Uhr in der Abreise-Zeitzone.

7. System nach einem der vorstehenden Ansprüche, worin das Westwärts-Dosisschema und/oder das Ostwärts-Dosisschema weiter 1-4 Dosen hellen Lichts zu verabreichen am Tag nach dem Ankunftstag umfasst.

8. System nach einem der vorstehenden Ansprüche, worin das Westwärts-Dosisschema und/oder das Ostwärts-Dosisschema weiter 1-4 Dosen hellen Lichts umfasst, zu verabreichen 2 Tage bzw. 3 Tage nach dem Ankunftstag.

9. Computer-implementiertes Verfahren zum Berechnen eines Schemas für Behandlungen mit hellem Licht, um beim Reisen beim Durchqueren einer Anzahl von Zeitzonen Jetlag-Symptome zu verhindern, wobei das Verfahren umfasst, Berechnen eines Schemas von Dosen hellen Lichts beginnend am Tag nach der Ankunft, worin jede Dosis durch ein Intervall von zwischen 2 und 4 Stunden, wie 3 Stunden, getrennt ist, worin
- beim Reisen westwärts der Zeitpunkt der ersten Dosis am Ankunftstag am Vormittag zwischen 11 und 13 Uhr, wie 12 Uhr, festgelegt wird, und
- beim Reisen ostwärts der Zeitpunkt der ersten Dosis am Ankunftstag um etwa x + 7 Uhr festgelegt wird, wenn weniger als 6 Zeitzonen überquert werden, und etwa um x Uhr festgelegt wird, wenn mindestens 6 Zeitzonen durchquert werden, wobei x die Anzahl an durchquerten Zeitzonen darstellt.

10. Verfahren nach Anspruch 9, wobei beim Reisen nach Westen der Zeitpunkt der ersten Dosis am zweiten Tag nach Ankunft zwischen 8 und 10 Uhr, wie 9 Uhr, festgelegt wird, wenn weniger als 7 Zeitzonen durchquert werden, und zwischen 11 und 13 Uhr, wenn mindestens 7 Zeitzonen durchquert wurden.

11. Verfahren nach Anspruch 9 bis 10, wobei beim Reisen nach Westen der Zeitpunkt der ersten Dosis am dritten Tag nach Ankunft zwischen 8 und 10 Uhr, wie 9 Uhr, festgelegt wird, und der Zeitpunkt der ersten Dosis am vierten Tag nach Ankunft zwischen 8 und 10 Uhr, wie 9 Uhr, festgelegt wird.

12. Verfahren nach einem der Ansprüche 9 bis 11, wobei beim Reisen nach Osten der Zeitpunkt der ersten Dosis am zweiten Tag nach Ankunft zwischen 8 und 11 Uhr, wie 9 Uhr oder 10 Uhr, festgelegt wird, und der Zeitpunkt der ersten Dosis am dritten Tag nach Ankunft zwischen 8 und 10 Uhr, wie 9 Uhr, festgelegt wird.

13. Verfahren nach einem der Ansprüche 9 bis 12, wobei beim Reisen nach Osten der Zeitpunkt der ersten Dosis am vierten Tag nach Ankunft zwischen 8 und 10 Uhr, wie 9 Uhr, festgelegt wird.

14. Verfahren nach einem der Ansprüche 9 bis 13, wobei die Anzahl täglicher Dosen 3 oder mindestens 4 ist und worin eine Dosis hellen Lichts 8-12 Minuten an hellem Licht entspricht, das über die Hörkanale verabreicht wird.

15. Computer-Programmprodukt mit einem Computer-lesbaren Medium, worin das Computerprogramm-Produkt geeignet ist, ein Behandlungs-Schema mit hellem Licht zu berechnen, um Jetlag-Symptome zu verhindern, wenn während des Reisens eine Anzahl an Zeitzonen durchquert werden, worin das Computer-Programmprodukt Mittel zur Durchführung aller Schritte des Verfahrens nach einem der Ansprüche 9 bis 14 umfasst, wenn in einem Daten-verarbeitenden System durchgeführt.

## Revendications

1. Système de prévention de symptômes de décalage horaire d'un utilisateur voyageant à travers des fuseaux horaires, ledit utilisateur ayant un temps de température quotidienne minimale Tₘᵢₙ, ledit système comprenant :
- une ou plusieurs unités de rayonnement aptes à diriger une lumière vive vers une ou plusieurs structures cérébrales neuroanatomiques dudit utilisateur à partir d'au moins une position non oculaire extracrânienne, et
- des moyens de planification pour calculer un schéma de dose vers l'ouest pour voyager vers l'ouest et un schéma de dose vers l'est pour voyager vers l'est, lesdits schémas de dose étant aptes à être appliqués au dit utilisateur le jour de son arrivée, dans lequel
-- le schéma de dose vers l'ouest comprend une ou plusieurs doses de lumière vive programmées jusqu'à six heures avant Tₘᵢₙ et aptes à augmenter la vigilance dudit utilisateur, et une ou plusieurs doses de lumière vive programmées dans les 4 heures avant Tₘᵢₙ et aptes à retarder Tₘᵢₙ dudit utilisateur, et
-- le schéma de dose vers l'est comprend :
--- lors de la traversée de moins de 6 fuseaux horaires : une dose de lumière vive programmée entre 1 heure et 4 heures après Tₘᵢₙ, ladite dose étant apte à avancer Tₘᵢₙ dudit utilisateur, et une pluralité de doses de lumière vive programmées plus de 6 heures après Tₘᵢₙ, chacune desdites doses étant apte à augmenter la vigilance dudit utilisateur, et
--- lors de la traversée d'au moins 6 fuseaux horaires : une pluralité de doses de lumière vive programmées entre 1 heure et 4 heures avant Tₘᵢₙ, chacune desdites doses étant apte à retarder Tₘᵢₙ dudit utilisateur.

2. Système selon la revendication 1, dans lequel deux doses consécutives de lumière vive sont séparées d'au moins 2 heures ou d'au moins 3 heures.

3. Système selon l'une quelconque des revendications précédentes, dans lequel le schéma de dose vers l'est comprend 4 doses de lumière vive programmées respectivement à 4 heures, 3 heures, 2 heures et 1 heure avant Tₘᵢₙ.

4. Système selon l'une quelconque des revendications précédentes, dans lequel le schéma de dose vers l'est comprend 4 doses de lumière vive programmées à 2 heures avant Tₘᵢₙ.

5. Système selon l'une quelconque des revendications précédentes, dans lequel la lumière vive est administrée à travers les conduits auditifs dudit utilisateur, et/ou dans lequel une dose de lumière vive correspond à 8 à 12 minutes de lumière vive.

6. Système selon l'une quelconque des revendications précédentes, dans lequel Tₘᵢₙ est obtenu en tant qu'entrée d'utilisateur ou dans lequel Tₘᵢₙ dudit utilisateur est prédéfini, par exemple prédéfini pour être à 3 heures, 3 heures 30, 4 heures, 4 heures 30 ou 5 heures dans le fuseau horaire de départ.

7. Système selon l'une quelconque des revendications précédentes, dans lequel le schéma de dose vers l'ouest et/ou le schéma de dose vers l'est comprennent en outre 1 à 4 doses de lumière vive programmées le lendemain du jour d'arrivée.

8. Système selon l'une quelconque des revendications précédentes, dans lequel le schéma de dose vers l'ouest et/ou le schéma de dose vers l'est comprennent en outre 1 à 4 doses de lumière vive programmées respectivement deux jours et trois jours après le jour d'arrivée.

9. Procédé mis en oeuvre par ordinateur pour calculer un planning de traitements par lumière vive pour la prévention de symptômes de décalage horaire lors de la traversée d'un certain nombre de fuseaux horaires lors d'un voyage, le procédé comprenant le calcul d'un planning de doses de lumière vive commençant le jour d'arrivée, deux doses successives étant séparées d'un intervalle entre 2 heures et 4 heures, par exemple 3 heures, dans lequel
- lors d'un voyage vers l'ouest, le moment de la première dose le jour d'arrivée est programmé dans la matinée entre 11 heures et 13 heures, par exemple 12 heures, et
- lors d'un voyage vers l'est, le moment de la première dose le jour d'arrivée est programmé à environ x + 7 heures si moins de six fuseaux horaires sont traversés, et à environ x heures si au moins six fuseaux horaires sont traversés, où x est le nombre de fuseaux horaires traversés.

10. Procédé selon la revendication 9, dans lequel, lors d'un voyage vers l'ouest, le moment de la première dose le deuxième jour après l'arrivée est programmé entre 8 et 10 heures, par exemple 9 heures, si moins de sept fuseaux horaires sont traversés, et entre 11 et 13 heures, par exemple 12 heures, si au moins sept fuseaux horaires sont traversés.

11. Procédé selon l'une quelconque des revendications 9 et 10, dans lequel, lors d'un voyage vers l'ouest, le moment de la première dose le troisième jour après l'arrivée est programmé entre 8 et 10 heures, par exemple 9 heures, et le moment de la première dose le quatrième jour après l'arrivée est programmé entre 8 et 10 heures, par exemple 9 heures.

12. Procédé selon l'une quelconque des revendications 9 à 11, dans lequel, lors d'un voyage vers l'est, le moment de la première dose le deuxième jour après l'arrivée est programmé entre 8 et 11 heures, par exemple 9 ou 10 heures, et le moment de la première dose le troisième jour après l'arrivée est programmé entre 8 et 10 heures, par exemple 9 heures.

13. Procédé selon l'une quelconque des revendications 9 à 12, dans lequel, lors d'un voyage vers l'est, le moment de la première dose le quatrième jour après l'arrivée est programmé entre 8 et 10 heures, par exemple 9 heures.

14. Procédé selon l'une quelconque des revendications 9 à 13, dans lequel le nombre de doses quotidiennes est égal à 3 ou à au moins 4 et dans lequel une dose de lumière vive correspond à 8 à 12 minutes de lumière vive administrée par l'intermédiaire des conduits auditifs d'un utilisateur.

15. Produit de programme informatique comportant un support lisible par ordinateur, ledit produit de programme informatique étant apte à calculer un planning de traitements par lumière vive pour la prévention de symptômes de décalage horaire lors de la traversée d'un certain nombre de fuseaux horaires lors d'un voyage, ledit produit de programme informatique comprenant des moyens pour effectuer toutes les étapes du procédé selon l'une quelconque des revendications 9 à 14, lorsqu'il est exécuté sur un système informatique.
